# EUROPEAN PATENT APPLICATION

(11) **EP 3 315 200 A1**
(43) Date of publication of application: **02.05.2018**
(21) Application number: 17382719.7
(22) Date of filing: 27.10.2017
(51) Int. Cl.: B01L 3/00, B01L 7/00, G01N 15/02

(54) **METHOD FOR EXTRACTING EXOSOMES AND MICROFLUIDIC DEVICE FOR EXTRACTING EXOSOMES**

(30) Priority: 27.10.2016 ES 201631380
(71) Applicant: Mondragon Goi Eskola Politeknikoa Jose Maria Arizmendiarrieta, S.COOP., 20500 Arrasate-Mondragon (ES); Asociación Centro de Investigación Cooperativa en Biociencias - CIC bioGUNE, 48160 Derio (ES)
(72) Inventor: BOUALI SAIDI, Mohammed Mounir, 20500 ARRASATE - MONDRAGON (ES); MARTIN MAYOR, Alain, 20500 ARRASATE - MONDRAGON (ES); AGINAGALDE UNANUE, Maialen, 20500 ARRASATE - MONDRAGON (ES); ERRARTE YARZA, Ane, 20500 ARRASATE - MONDRAGON (ES); ELORTZA BASTERRIKA, Felix Roberto, 48160 DERIO (ES); FALCON PEREZ, Juan Manuel, 48160 DERIO (ES); GONZALEZ JIMENEZ, Maria Esperanza, 48160 DERIO (ES); ILORO MANZANO, Ibon, 48160 DERIO (ES)
(74) Representative: Igartua, Ismael

(57) **Abstract**

Method for extracting exosomes depending on their diameter, comprising a step of introducing a fluid sample comprising at least two exosome populations, each with a different diameter, in a microfluidic device (10) comprising at least one channel (1) comprising at least one inlet (2) for the fluid sample and at least two openings (3) distributed along the channel (1) allowing the exosome populations to move out and an outlet (5) for the remaining fluid sample, and means (6) for generating a temperature gradient in the channel (1); and a step in which the fluid sample is made to flow through the channel (1) while a temperature gradient is applied on the channel, causing each population to move out through a different opening (3). The present invention also relates to a microfluidic device (10) for the application of the method.

## Description

### TECHNICAL FIELD

The present invention relates to methods relating to the extraction of biological particles present in a fluid sample and microfluidic devices for extracting said biological particles.

### PRIOR ART

Exosomes are small vesicles that cells produce and secrete into extracellular medium containing relevant information (nucleic acids, lipids, metabolites, peptides, proteins, etc.) about the origin and state of said cells. These small vesicles have been extracted from various biological fluids due to the tremendous interest thereof as a biological source for identifying and defining non-invasive biomarkers for diseases. Furthermore, these biological particles are directly involved in the development of various pathologies including cancer, metabolic diseases and cardiovascular diseases.

Nowadays exosome isolation and purification is a method consisting of several tedious steps which makes it difficult for these investigations to progress towards a clinical application.

Microfluidic systems that are used to detect, capture, separate and enrich particles suspended or dispersed in a fluid are known in the prior art. By way of example, US20140030788A1 describes a method and device for separating three populations of biological particles, each population with different diameters, in which the sample is introduced in a fluid path comprising obstacles retaining one of the populations and allowing the flow of the rest of the populations based on the porosity of the obstacles.

WO2015139019A1 also describes a microfluidic system in which exosomes are captured by means of these exosomes binding to magnetic microbeads.

### DISCLOSURE OF THE INVENTION

The object of the invention is to provide an extraction method for extracting exosomes and a microfluidic extraction device for extracting exosomes, as defined in the claims.

One aspect of the invention relates to a method for extracting exosomes depending on their diameter, comprising the following steps:
- introducing a fluid sample comprising at least two exosome populations, each with a different diameter, in a microfluidic device comprising:
   - at least one channel comprising at least one inlet for the fluid sample and at least two openings distributed along the channel allowing the exosome populations to move out and an outlet for the remaining fluid sample, and
   - means for generating a temperature gradient in the channel; and
- making the fluid sample flow through the channel while a temperature gradient is applied on the channel, causing each population to move out through a different opening.

Another aspect of the invention relates to a microfluidic device for extracting exosomes depending on their diameter, comprising:
- at least one channel comprising at least one inlet for the fluid sample and at least two openings distributed along the channel allowing an exosome population to move out and an outlet for the remaining fluid sample; and
- means for generating a temperature gradient, causing each population to move out through a different opening.

The method and device of the invention allow extracting exosomes based on diameter in a simpler and less expensive manner compared to the prior art, since the central channel does not have obstacles, nor does it require using reagents such as exosome-specific binding elements.

Additionally, the exosomes are extracted at the moment in which the first exosome population moves out of the opening of the channel, without having to wait for the entire sample to flow through the channel. This allows having control over exosome population separation and extraction during sample processing and not at the end thereof. This control is extremely important for processes of extracting large volumes of fluid samples under constant flow, in which the process is simplified, downtimes are reduced and a better process yield is obtained.

These and other advantages and features of the invention will become evident in view of the drawings and the detailed description of the invention.

### DESCRIPTION OF THE DRAWINGS

Figure 1 shows a diagram of the microfluidic device according to a first embodiment of the invention.
Figure 2 shows a diagram of the microfluidic device according to a second embodiment of the invention.
Figure 3 shows a diagram of the microfluidic device according to a third embodiment of the invention.
Figure 4 shows a diagram of the microfluidic device according to a fourth embodiment of the invention.

### DETAILED DISCLOSURE OF THE INVENTION

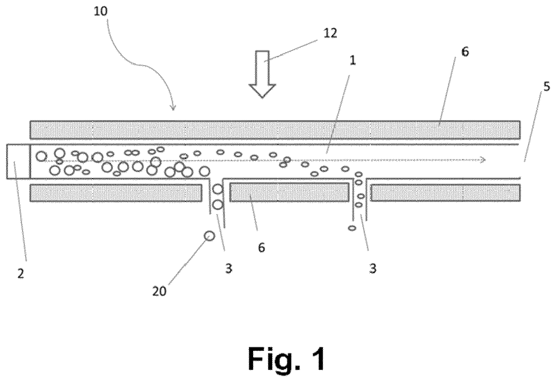

The method for extracting exosomes of the invention comprises the following steps:
- introducing a fluid sample comprising at least two exosome populations, each with a different diameter, in a microfluidic device 10 such as the one shown, for example, in Figure 1, which comprises:
   - at least one channel 1 comprising at least one inlet 2 for the fluid sample and at least two openings 3 distributed along the channel 1 allowing the exosome populations to move out and at least one outlet 5 for the remaining fluid sample, and
   - means 6 for generating a temperature gradient in the channel 1; and
- making the fluid sample flow through the channel 1 while a temperature gradient is applied on the channel 1, causing each population to move out through a different opening 3.

In the context of the invention, exosome is understood to be a lipid- and/or protein-based microparticle or nanoparticle present in a sample, for example, a biological fluid obtained from a subject or a cell, bacterial or fungal culture. The term "exosome" also refers to structures of a synthetic or biological origin formed by one or more lipid layers which can contain different proteins, nucleic acids, lipids, metabolites, and/or drugs both on their surface and inside same. These exosomes can be extracellular vesicles, microvesicles, nanovesicles, argosomes, ectosomes, dexosomes, apoptotic bodies and synthetic vesicles. The diameter or size of the exosomes can range between 20 nm and 5000 nm, depending on their origin.

In the context of the invention, exosome population is understood to be a group of exosomes having a specific range of diameter or size.

The method of the invention allows extracting exosome populations with diameters or sizes that are different from one another by at least 50 nm. This difference also applies to differences in hydrodynamic diameters that reach at least 50 nm.

The fluid sample comprising the exosome populations refers to a plant or animal biological fluid sample, such as a blood, serum, urine, saliva, tear, vaginal discharge, seminal discharge, cerebrospinal fluid, brain fluid, sweat, sputum, cervical wash, respiratory tract secretion, intestinal secretion, synovial secretion, and amniotic fluid sample; or a fluid, preferably a liquid fluid in which different exosome populations have been dispersed.

Depending on the nature of the fluid sample, as well as the composition thereof, it is suitable to first treat said sample before subjecting it to the method of the invention.

Examples of prior treatment of the fluid sample may include: the enrichment of exosomes or particles in the sample which favor the extraction of the exosome populations of interest, fluid sample dilution or elimination of impurities which affect exosome extraction, among others.

In the context of the invention, microfluidic device 10 is understood to be that device which uses volumetric liquid flows in the range of nanoliters or microliters per minute in a processor, also known as chip-based microfluidic systems.

As has already been indicated in reference to Figure 1, the microfluidic device 10 of the invention comprises a channel 1. In the context of the invention, the channel 1 is a passage through which the sample flows. In a preferred embodiment, the channel 1 is a conduit. This channel 1 may or may not be covered.

The channel 1 has dimensions according to the sample processing conditions: the flow rate, the temperature gradient, the exosome concentration and diameter or size and the fluid composition.

In a particular embodiment, the channel 1 has a depth at least five times the height and a length at least six times the depth. These dimensions allow generating stable streamlines along the channel 1.

By way of example, the channel 1 has a height between about 1600 µm and 20 µm, preferably 1600 µm, 800 µm, 400 µm, 200 µm, 100 µm, 50 µm or 20 µm; a depth between 8000 µm and 100 µm, and a length between 0.6 mm and 48 mm.

The channel 1 of the invention can be made with any polymeric, ceramic and/or metallic material, preferably with materials that are good thermal conductors and compatible with the samples to be processed.

By way of non-limiting example, a polymeric material can be a polyolefin, such as cyclic olefin copolymer, low- or high-density polyethylene, polypropylene or ethylenepropylene rubber.

By way of non-limiting example, a ceramic material can be beryllium oxide, graphite, diamond, borosilicate glass or silicon dioxide.

By way of non-limiting example, a metallic material can be stainless steel, silver, cobalt, chromium or titanium alloys.

In a preferred embodiment, the channel 1 comprises a cyclic olefin copolymer.

The fluid sample can sometimes contain interfering elements for the extraction that should be retained in the channel 1. By way of non-limiting example, the interfering elements can be: metabolites, fatty acids or protein aggregates. Therefore, in a particular embodiment the inner face of the channel 1, with which the fluid sample comes into contact, comprises elements for retaining these interfering elements, where these elements can be physical, chemical or biological elements.

In a preferred embodiment, the inlet 2 and outlet 5 of the channel 1 are located at the beginning and at the end of the channel 1, respectively. In a preferred embodiment, the inlet and/or outlet are arranged in an axial direction of the channel 1. In another embodiment in which the channel is a covered conduit, the inlet and/or outlet are arranged in a direction orthogonal to the direction of the fluid of the sample.

The channel 1 further comprises openings 3 through which the exosome populations 20 move out as they approach said openings 3.

The openings 3 are ports or conduits connected to a specific reservoir in which the extracted exosomes accumulate. These ports or conduits can also be connected to another measuring device or instrument. By way of example, this device or instrument detects and/or quantifies the exosomes or particles for control that are extracted during the extraction process.

If the opening is a conduit, in a particular embodiment, it is arranged at an angle between 30° and 90° with respect to the flow direction of the fluid sample, preferably 60°.

The openings 3 can have the same or different sizes. Regardless of whether these openings are the same or different, the maximum size of the opening is preferably half the height of the channel 1 or of the branch in the event of a branched channel 1.

The number of openings 3 can vary depending on the number of populations 20 to be extracted.

As shown in Figure 1, in a preferred embodiment these openings 3 are arranged in an aligned manner along the channel 1.

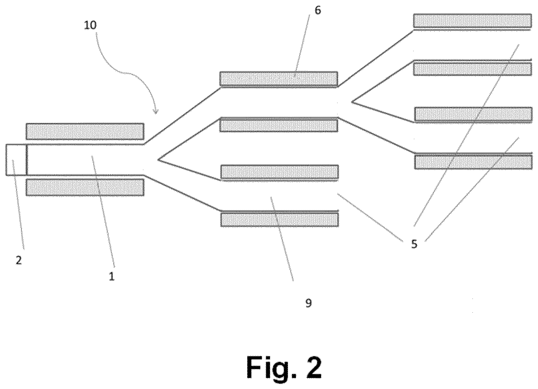

In another embodiment as shown in Figure 2, the central channel is branched, wherein the openings 3 are arranged along each branch 9 or at the distal end of the branch 9, coinciding with the outlet 5 of the branch.

The openings 3 are separated from one another and the separating distance is set depending on the requirements of each case, by way of example, the channel height, the gradient applied, the flow applied and/or the diameter of the exosome populations to be extracted.

The sample is moved in and out and made to flow using means known by the person skilled in the art.

The temperature of the fluid sample at the moment of being introduced in the channel 1 can vary depending on the nature of the sample and on the temperature gradient to be applied. In a preferred embodiment, the sample at the moment of being introduced in the channel 1 has a temperature that is less damaging for the exosome, this temperature being between 4°C and 37°C, preferably between 5°C and 15°C.

As shown in Figures 1 to 3, the microfluidic device comprises means for generating the temperature gradient 12.

The means for generating the temperature gradient 12 can be, in a non-limiting manner, a thermostatic bath, a Peltier system or electrodes.

Depending on the nature of said means, they can be arranged in contact with the channel 1 or on the channel 1. In turn, these means can be arranged along the channel 1 or in specific areas of the channel 1 in which the temperature gradient is to be generated.

In a preferred embodiment, the temperature gradient 12 is generated through electrodes.

This temperature gradient 12 can be applied at different angles with respect to the flow direction of the sample.

In a preferred embodiment, the temperature gradient 12 is perpendicular to the flow direction of the sample.

In a preferred embodiment, the temperature gradient perpendicular to the generated flow direction is at least 0.5°C.

The exosomes tend to move to the area of the channel 1 in which the temperature of the gradient is lower, so in a preferred embodiment, the openings 3 are arranged in said area. The exosomes having the largest diameter spread out and move to the area with a lower temperature before the exosomes having the next largest diameter do, so the first exosome population to move out is the one having the largest diameter, followed by the rest of the populations in the order from the largest to the smallest diameter.

It is suitable to modulate the temperature gradient along the channel 1 depending on the exosome origin and size, the fluid composition, the dimensions and shape of the channel 1 and the flow rate; therefore in a particular embodiment, the temperature gradient is variable along the channel 1.

In the event of a branched channel 1 as shown in Figure 2, the means 6 generating the temperature gradient are arranged in the channel 1 and in each branch 9, where different temperature gradients can be generated in each branch of the channel 1.

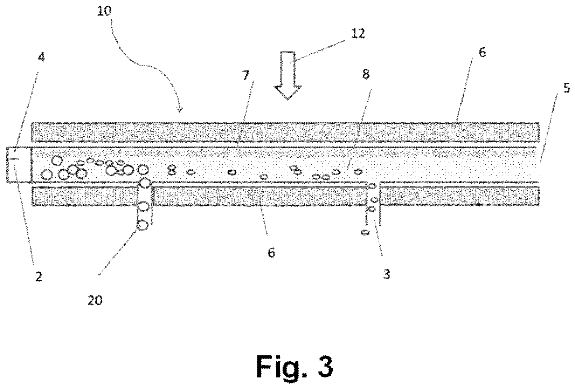

In a particular embodiment as shown in Figure 3, the channel 1 comprises at least one additional inlet 4 through which a fluid 7 is introduced, together with the fluid sample, in the channel 1 in order to move said fluid sample to an area of interest 8. The exosomes are therefore introduced in a more limited area corresponding to the area of interest 8 of the channel 1, and the gradient therefore more uniformly affects all the exosome populations.

The fluid 7 can be, in a non-limiting manner, an aqueous buffer. In one embodiment of the invention, the fluid 7 is saline phosphate buffer.

In another particular embodiment, the channel 1 comprises at least a second additional inlet 4 through which a second fluid 7 is introduced in the channel 1 together with the fluid sample; the sample is thereby introduced in the channel enveloped in a first and second fluid 7. Like in the preceding embodiment, the exosomes are introduced in a more limited area corresponding to the area of interest 8 of the channel 1, and the gradient therefore more uniformly affects all the exosome populations.

The first and second fluid 7 can be the same or different depending on the exosome origin and size, the fluid composition, and the dimensions and shape of the channel 1.

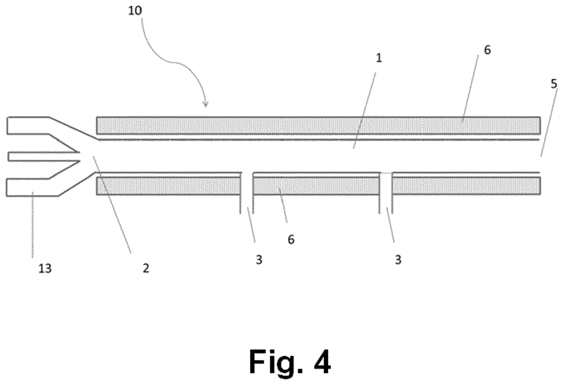

In a particular embodiment, the fluid sample is introduced enveloped in the fluid 7. By way of a non-limiting example, the fluid sample moves in through a flow cytometer 13 which is connected to the inlet 2 of the channel 1. The flow cytometer 13 comprises two or three inlets, depending on whether the sample is to be introduced covered on one side or enveloped in the fluid 7. If the sample is enveloped as shown in Figure 4, the cytometer 13 comprises three inlets; the fluid 7 is introduced from the upper and lower inlets and the fluid sample is introduced from the inlet in the center. The use of cytometer simplifies the structure of the device as it requires only one inlet 2 of the channel 1 for introducing the sample enveloped in or covered by the fluid 7 to be introduced therein.

In the embodiments in which the fluid sample is introduced enveloped in or covered by the fluid 7, the flow rate of each may vary. In a preferred embodiment, the fluid sample is introduced enveloped in a fluid 7, wherein the inflow rate of the fluid sample is 40 times lower than the total inflow rate.

Depending on the nature and concentration of the exosome populations present in the fluid sample, said sample can be subjected to more than one cycle of the method of the invention, until the complete extraction of the exosome populations. This cycle repetition can be performed on a single device of the invention or on several interconnected devices of the invention.

Additionally, in a particular embodiment the fluid sample can contain particles for control of the extraction method such that they allow detecting *in situ* that the extraction method is being performed correctly.

In a preferred embodiment, the particles for control are particles having physical characteristics comparable to the exosome populations to be extracted. These physical characteristics, such as the size, weight, etc., cause the control particles to have a behavior that is similar or comparable to the exosome population to be extracted.

In a preferred embodiment, these particles can furthermore be identified and/or differentiated from the exosome populations to be extracted. For example, one way of identifying and differentiating them from exosomes would be to label them for subsequent detection.

A second aspect of the invention relates to a microfluidic device 10 configured for extracting exosomes depending on their diameter, as described above in reference to the extraction method.

In a particular embodiment, the channel 1 is divided into parts that can be put together, each part having at least one opening for a specific exosome population diameter, the channel 1 being configured with the required parts according to the type of fluid sample and/or the number of exosome populations to be separated.

In a particular embodiment, the microfluidic device further comprises a flow cytometer according to the features described above, through which the fluid sample and the fluid 7 are introduced in the channel 1.

Both the method and the microfluidic device of the invention are used for extracting exosomes depending on their diameter. This extraction can have many purposes or uses, such as the complete removal of exosomes from a fluid sample, the concentration of the fluid sample in an exosome of a specific size in which the exosomes to be extracted will be those that are not desired in the fluid sample, or for the diagnosis of diseases by means of isolating and detecting exosomes which are biomarkers for said diseases.

## Claims

1. Method for extracting exosomes depending on their diameter, **characterized in that** it comprises the following steps:
- introducing a fluid sample comprising at least two exosome populations each with a different diameter in a microfluidic device (10) comprising:
- at least one channel (1) comprising at least one inlet (2) for the fluid sample and at least two openings (3) distributed along the channel (1) allowing the exosome populations to move out and an outlet (5) for the remaining fluid sample, and
- means (6) for generating a temperature gradient in the channel (1); and
- making the fluid sample flow through the channel (1) while a temperature gradient is applied on the channel (1), causing each population to move out through a different opening (3).

2. Method for extracting exosomes according to claim 1, wherein the temperature gradient is perpendicular to the flow direction of the fluid sample.

3. Method for extracting exosomes according to claim 1 or 2, wherein a temperature gradient of at least 0.5°C is generated.

4. Method for extracting exosomes according to any of the preceding claims, wherein the openings (3) are arranged in the area of the channel (1) in which the temperature of the gradient is lower.

5. Method for extracting exosomes according to any of the preceding claims, wherein the temperature gradient is variable along the channel (1).

6. Method for extracting exosomes according to any of the preceding claims, wherein the openings (3) are arranged in an aligned manner along the channel (1).

7. Method for extracting exosomes according to any of the preceding claims, wherein the channel (1) comprises at least one additional inlet (4) through which a fluid (7) is introduced together with the fluid sample in the channel (1) in order to move said fluid sample to an area of interest (8).

8. Method for extracting exosomes according to any of claims 1 to 6, wherein the fluid sample is introduced enveloped in a second fluid (7).

9. Method for extracting exosomes according to any of the preceding claims, wherein the fluid sample comprises particles for control of the extraction method, said particles preferably having physical characteristics comparable to the exosome populations to be extracted.

10. Method for extracting exosomes according to claim 9, wherein the particles for control can be identified and/or differentiated from the exosome populations to be extracted.

11. Microfluidic device for extracting exosomes depending on their diameter, **characterized in that** it comprises:
- at least one channel (1) comprising at least one inlet (2) for the fluid sample and at least two openings (3) distributed along the channel (1) allowing the exosome populations to move out and an outlet (5) for the remaining fluid sample; and
- means (6) for generating a temperature gradient in the channel (1), causing each population to move out through a different opening (3).

12. Microfluidic device for extracting exosomes according to claim 11, wherein the temperature gradient is perpendicular to the flow direction in the channel (1).

13. Microfluidic device for extracting exosomes according to claim 11 or 12, wherein the openings (3) are arranged in the area of the channel (1) in which the temperature of the gradient is lower, the openings (3) preferably being arranged in an aligned manner along the channel (1).

14. Microfluidic device for extracting exosomes according to any of claims 11 to 13, wherein the channel (1) comprises at least one additional inlet (4) through which a fluid is introduced together with the fluid sample in the channel (1) in order to move said fluid sample to an area of interest (8).

15. Microfluidic device for extracting exosomes according to any of claims 11 to 14, wherein the channel (1) is divided into parts that can be put together, each part having at least one opening for an exosome population of a specific diameter, the channel (1) being configured with the parts required according to the type of fluid sample and/or the number of exosome populations to be separated.
